# EUROPÄISCHE PATENTSCHRIFT

(11) **EP 0 729 454 B1**
(45) Veröffentlichungstag und Bekanntmachung des Hinweises auf die Patenterteilung: **31.03.1999**
(21) Anmeldenummer: 95901381.4
(22) Anmeldetag: 15.11.1994
(51) Int. Cl.: C07D 201/08

(54) **VERFAHREN ZUR HERSTELLUNG VON CAPROLACTAM**
PROCESS FOR MANUFACTURING CAPROLACTAM
PROCEDE DE PREPARATION DE CAPROLACTAME

(30) Priorität: 20.11.1993 DE 4339648
(43) Veröffentlichungstag der Anmeldung: 04.09.1996
(73) Patentinhaber: BASF Aktiengesellschaft, 67063 Ludwigshafen (DE)
(72) Erfinder: FUCHS, Eberhard, D-67227 Frankenthal (DE); WITZEL, Tom, D-67069 Ludwigshafen (DE)
(86) Internationale Anmeldenummer: EP9403782
(87) Internationale Veröffentlichungsnummer: WO9514665

(56) Entgegenhaltungen:
- EP-A- 0 150 295
- FR-A- 2 029 540
- US-A- 2 301 964
- US-A- 4 625 023
- US-A- 4 628 085

## Beschreibung

Die vorliegende Erfindung betrifft ein neues Verfahren zur Herstellung von cyclischen Lactamen durch Umsetzung von Aminocarbonsäurenitrilen mit Wasser in Gegenwart von Katalysatoren.

Aus der US-A 4 628 085 ist die Umsetzung von 6-Aminocapronsäurenitril mit Wasser in der Gasphase an saurem Kieselgel bei 300°C bekannt. Als Produkt der quantitativ verlaufenden Umsetzung wird mit einer anfänglichen Selektivität von 95 % Caprolactam erhalten, doch ist ein schneller Produktivitäts- und Selektivitätsrückgang festzustellen. Ein ähnliches Verfahren wird in der US-A 4 625 023 beschrieben, nach der ein hochverdünnter Gasstrom aus 6-Aminocapronsäurenitril, Adipinsäuredinitril, Ammoniak, Wasser und Trägergas über ein Kieselgel- und ein Kupfer/Chrom/Barium-Titanoxid Katalysatorbett geleitet werden. Bei 85 % Umsatz wird Caprolactam mit einer Selektivität von 91 % erhalten. Auch hier ist eine schnelle Katalysatordesaktivierung zu beobachten.

Gegenstand der US-A 2 301 964 ist die nicht katalysierte Umsetzung von 6-Aminocapronsäurenitril zu Caprolactam in wäßriger Lösung bei 285°C. Die Ausbeuten liegen unter 80 %.

Die FR-A 2 029 540 beschreibt ein Verfahren zur Cyclisierung von 6-Aminocapronitril zu Caprolactam mittels Katalysatoren, wobei als Katalysatoren, wobei als Katalysatoren metallisches Zn oder Cu-Pulver oder Oxide, Hydroxide, Halogenide, Cyanide des Rubidiums, Bleis, Quecksilbers oder der Elemente mit einer Ordnungszahl 21 bis 30 oder 39 bis 48 Verwendung finden. Die beschriebenen Katalysatoren werden in diskontinuierlich betriebenen Rührautoklaven als Suspensionskatalysatoren eingesetzt. Caprolactam wird dabei in Ausbeuten bis zu 83 % erhalten. Die vollständige Abtrennung der Katalysatoren vom Wertprodukt Caprolactam bereitet jedoch Probleme, da Caprolactam mit den löslichen Bestandteilen der verwendeten Metalle Verbindungen bilden kann oder Feinstpartikel durch mechanisches Rühren entstehen können.

Aufgabe der vorliegenden Erfindung war es daher, ein Verfahren zur Herstellung cyclischer Lactame durch Umsetzung von Aminocarbonsäurenitrilen mit Wasser in Gegenwart von Katalysatoren zur Verfügung zu stellen, das die vorstehend beschriebenen Nachteile nicht mit sich bringt, hohe Ausbeuten und Selektivitäten liefert und eine kontinuierliche Fahrweise erlaubt.

Des weiteren sollte der Verbrauch am Katalysator so gering wie möglich gehalten werden. Ferner sollten die bei Suspensionsfahrweise auftretenden Abtrennprobleme, sei es durch Komplexbildung der löslichen Bestandteile des Katalysators mit Komponenten der Reaktionsmischung oder durch Feinstpartikel, die durch die starke mechanische Belastung beim Rührvorgang anfallen, vermieden werden.

Diese Aufgabe wird erfindungsgemäß dadurch gelöst, daß die Umsetzung in flüssiger Phase in einem Festbettreaktor in Gegenwart heterogener Katalysatoren, die unter den Reaktionsbedingungen keine löslichen Bestandteile aufweisen, durchgeführt wird. Die heterogenen Katalysatoren sind in einem Festbett angeordnet, das in Riesel- oder Sumpffahrweise kontinuierlich von dem Reaktionsgemisch durchströmt wird.

Bevorzugte Ausführungsformen des erfindungsgemäßen Verfahrens sind den Unteransprüchen zu entnehmen.

Als Ausgangsstoffe im erfindungsgemäßen Verfahren werden Aminocarbonsäurenitrile, vorzugsweise solche der allgemeinen Formel I 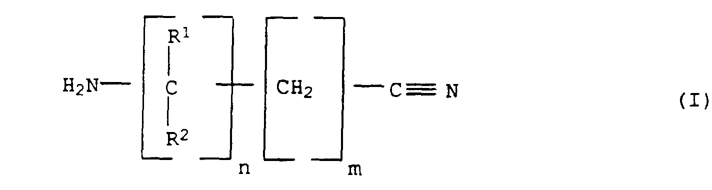 eingesetzt, wobei n und m jeweils die Werte 0, 1, 2, 3, 4, 5, 6, 7, 8 und 9 haben können und die Summe aus n + m mindestens 3, vorzugsweise mindestens 4 beträgt.

R¹ und R² können prinzipiell Substituenten jeglicher Art sein, wobei lediglich sichergestellt sein sollte, daß die gewünschte Cyclisierungsreaktion durch die Substituenten nicht beeinflußt wird. Vorzugsweise sind R¹ und R² unabhängig voneinander C₁-C₆-Alkyl- oder C₅-C₇-Cycloalkylgruppen oder C₆-C₁₂-Arylgruppen.

Besonders bevorzugte Ausgangsverbindungen sind Aminocarbonsäurenitrile der allgemeinen Formel

H₂N―(CH₂)ₘ―C≡N

wobei m einen Wert von 3, 4, 5 oder 6, insbesondere 5 aufweist. Für m = 5 ergibt sich als Ausgangsverbindung 6-Aminocapronsäurenitril.

Nach dem erfindungsgemäßen Verfahren werden die vorstehend beschriebenen Aminocarbonsäurenitrile mit Wasser in flüssiger Phase unter Verwendung heterogener Katalysatoren zu cyclischen Lactamen umgesetzt. Bei Verwendung von Aminocarbonsäurenitrilen der Formel I erhält man die entsprechenden cyclischen Lactame der Formel II 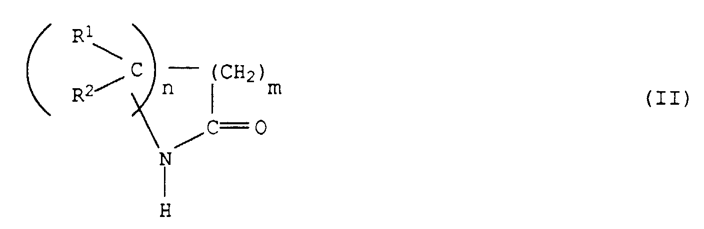 wobei n, m, R¹ und R² die vorstehend genannte Bedeutung haben. Besonders bevorzugte Lactame sind solche, in denen n = O ist und m einen Wert von 4,5 oder 6 hat, insbesondere 5 (im letzteren Fall erhält man Caprolactam).

Die Umsetzung wird in flüssiger Phase bei Temperaturen von im allgemeinen 140 bis 320°C, vorzugsweise 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.

Pro mol Aminocarbonsäurenitril werden im allgemeinen mindestens 0,01 mol, vorzugsweise 0,1 bis 20 und insbesondere 1 bis 5 mol Wasser eingesetzt.

Vorteilhaft wird das Aminocarbonsäurenitril in Form einer 1 bis 50 gew.-%igen, insbesondere 5 bis 50 gew.-%igen, besonders vorzugsweise 5 bis 30 gew.-%igen Lösung in Wasser (wobei dann das Lösungsmittel gleichzeitig Reaktionspartner ist) oder in Wasser/ Lösungsmittel-Gemischen eingesetzt. Als Lösungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer Lösungsmittel Anwendung finden. Mischungen aus Wasser und Alkanolen im Gewichtsverhältnis Wasser/Alkanol 1-75/25-99, vorzugsweise 1-50/50-99 haben sich in einigen Fällen als besonders vorteilhaft herausgestellt.

Es ist prinzipiell genauso möglich, die Aminocarbonsäurenitrile als Reaktand und gleichzeitig Lösungsmittel anzuwenden.

Als heterogene Katalysatoren können beispielsweise verwendet werden: Saure, basische oder amphotere Oxide der Elemente der zweiten, dritten oder vierten Hauptgruppe des Periodensystems, wie Calciumoxid, Magnesiumoxid, Boroxid, Aluminiumoxid, Zinn-Oxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titanoxid, amorph, als Anatas oder Rutil, Zirkonoxid, Zinkoxid, Manganoxid oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:

Vanadiniumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin sind Zeolithe, Phosphate und Heteropolysäuren, sowie saure und alkalische Ionenaustauscher wie beispielsweise Naphion als geeignete Katalysatoren zu nennen.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Die Katalysatoren können je nach der Zusammensetzung des Katalysators als Vollkontakt oder Trägerkatalysator verwendet werden. So kann z.B. Titan-Dioxid als Titan-Dioxid-Strang oder als auf einen Träger in dünner Schicht aufgebrachtes Titandioxid eingesetzt werden. Zum Aufbringen von TiO₂ auf einen Träger wie Siliciumdioxid, Aluminiumoxid oder Zirkondioxid sind alle in der Literatur beschriebenen Methoden verwendbar. So kann eine dünne TiO₂-Schicht durch Hydrolyse von Ti-Organylen wie Ti-Isopropylat oder Ti-Butylat, oder durch Hydrolyse von TiCl₄ oder anderen anorganischen Ti-haltigen Verbindungen aufgebracht werden. Auch Titanoxid-haltige Sole sind verwendbar.

Der Vorteil der erfindungsgemäßen Festbettfahrweise liegt einerseits in der Möglichkeit, die Cyclisierung auf einfache Weise kontinuierlich zu betreiben. Andererseits sind überraschenderweise die erzielten Ausbeuten und Selektivitäten im Festbett sehr hoch und erlauben somit kurze Verweilzeiten mit sehr hohen Durchsätzen. Da die verwendeten Katalysatoren nach bisherigen Beobachtungen eine hohe Lebensdauer aufweisen, ergibt sich ein extrem geringer Katalysator-Verbrauch. Die bei der Suspensionsfahrweise auftretenden Abtrennprobleme, sei es durch Komplexbildung der löslichen Bestandteile des Katalysators mit Komponenten der Reaktionsmischung oder durch Feinstpartikel, die durch die starke mechanische Belastung beim Rührvorgang anfallen, entfallen durch die kontinuierlich betriebene Festbettfahrweise vollständig.

### Beispiele

In einen geheizten Rohrreaktor von 25 ml Inhalt (Durchmesser 6 mm; Länge 800 mm), der mit Titandioxid (Anatas) in Form von 1,5 mm Strängen gefüllt war, wurde bei 100 bar eine Lösung von 6-Aminocapronsäurenitril (ACN) in Wasser und Ethanol in den in der Tabelle angegebenen Gewichtsverhältnissen geleitet. Der den Reaktor verlassende Produktstrom wurde gaschromatographisch und hochdruckflüssigchromatographisch (HPLC) analysiert. Die Ergebnisse sind ebenfalls der Tabelle zu entnehmen.

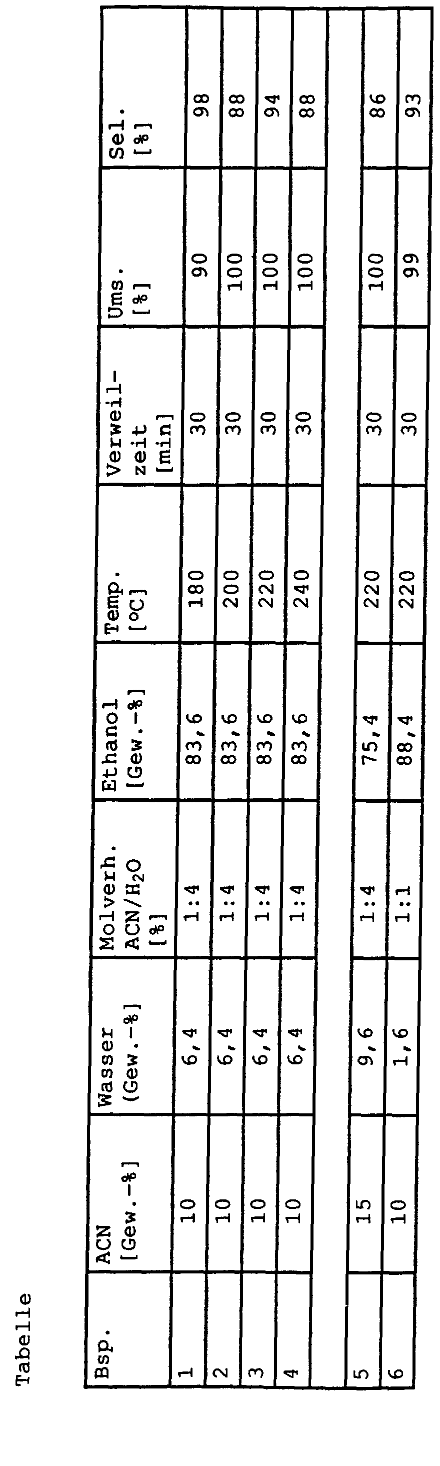

### Vergleichsversuch

Entsprechend den in Beispiel 1 beschriebenen Versuchen wurde eine Lösung von 10 % Aminocapronsäurenitril. 6,4 % Wasser und 83,6 % Ethanol ohne heterogenen Katalysator bei 250°C und einer Verweilzeit von 30 min in einem leeren Rohrreaktor umgesetzt. Der Umsatz betrug 28 % und die Selektivität zu Caprolactam 74 %.

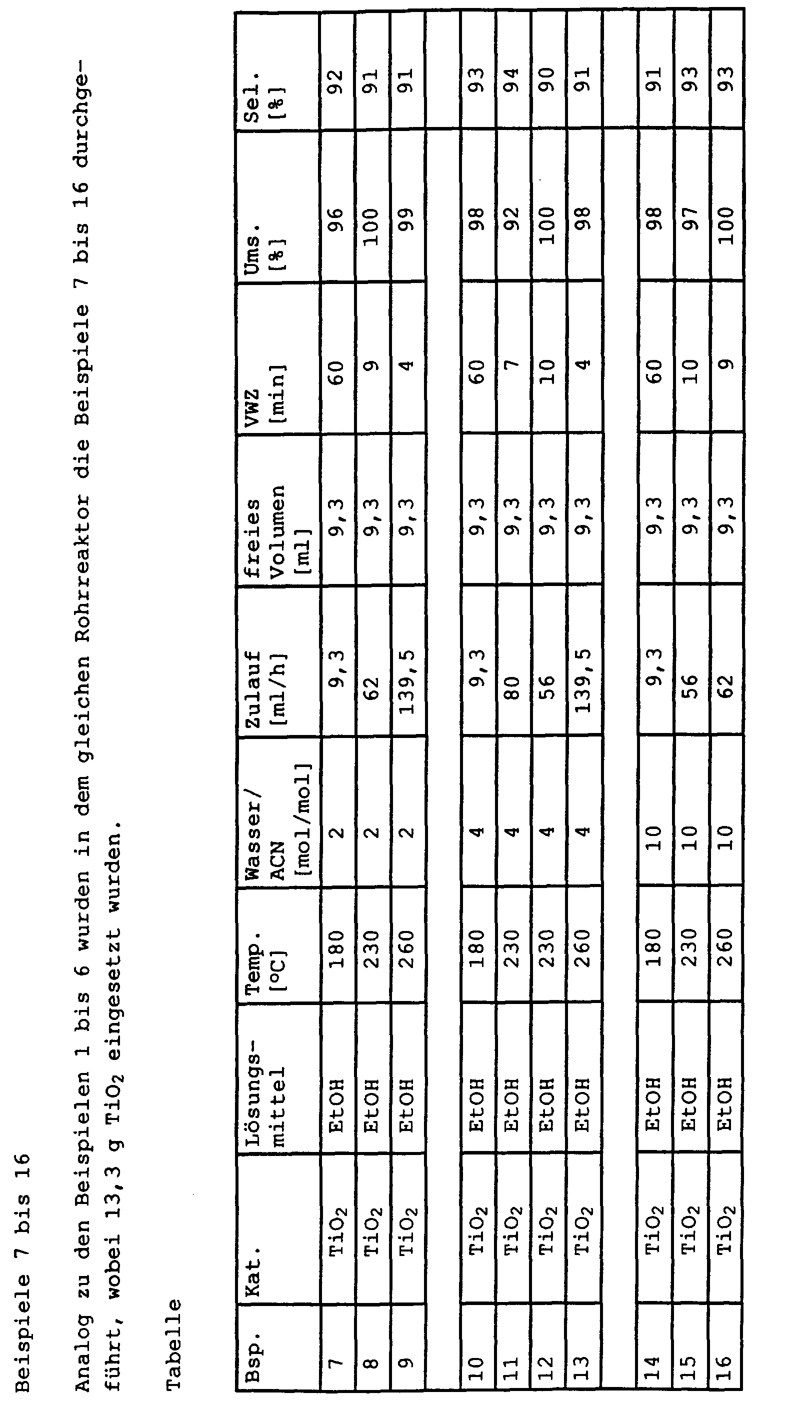

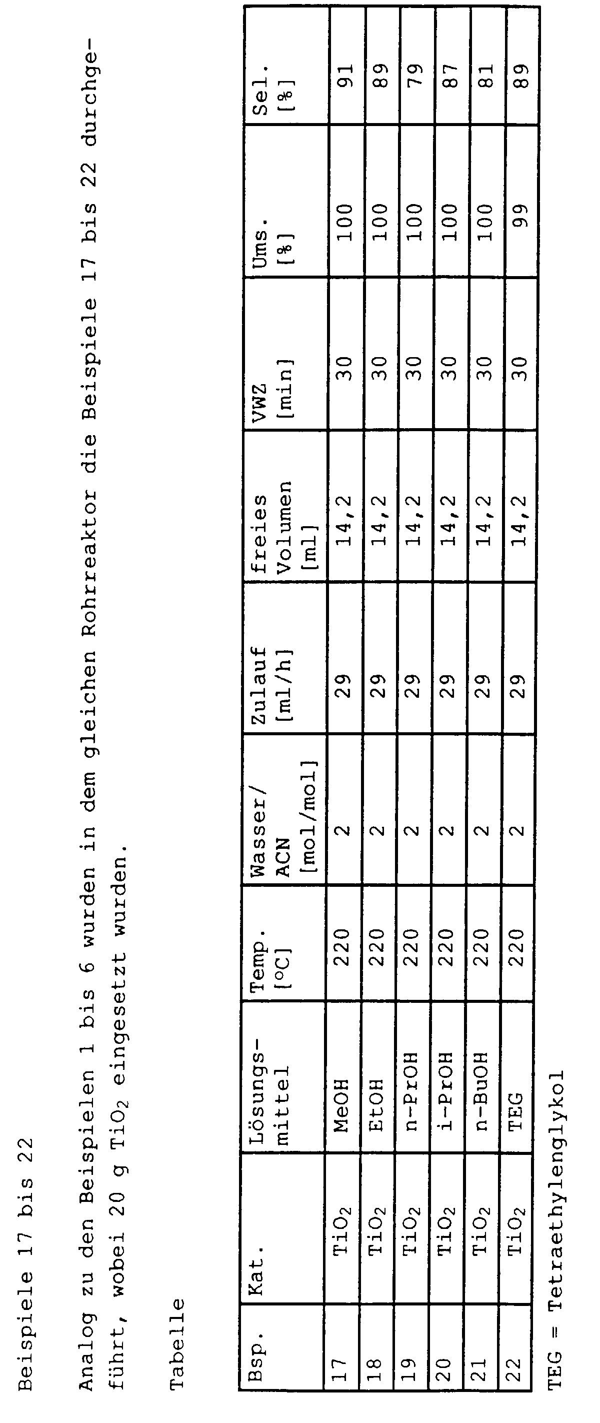

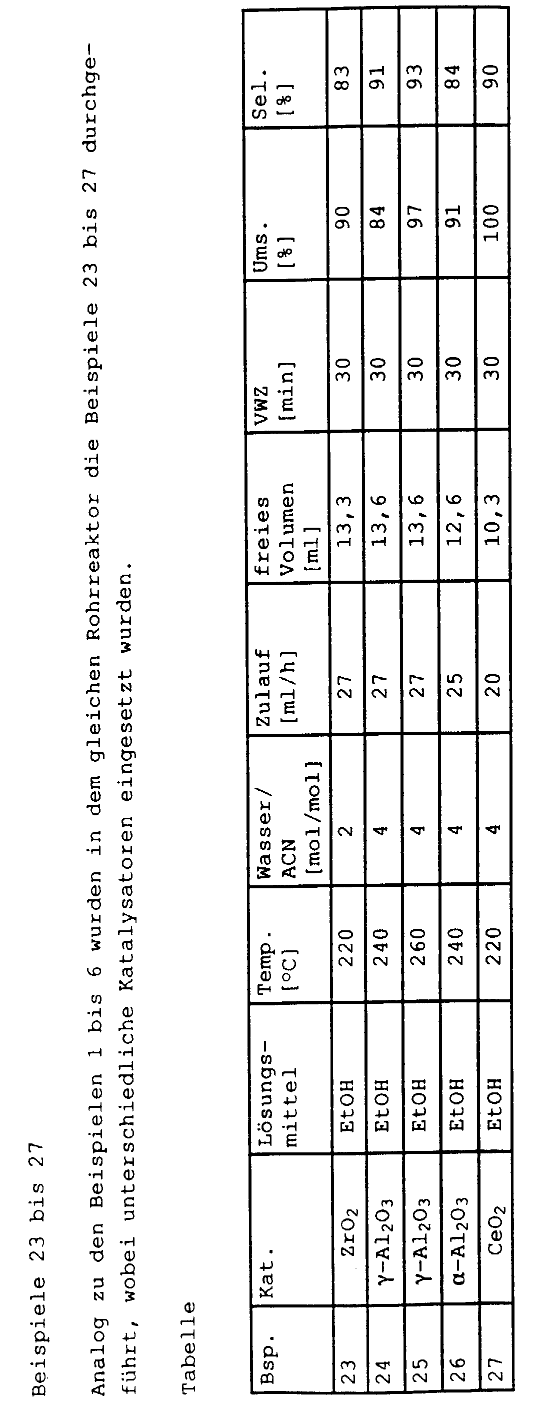

## Patentansprüche

1. Verfahren zur Herstellung cyclischer Lactame durch Umsetzung von Aminocarbonsäurenitrilen mit Wasser in Gegenwart von Katalysatoren, dadurch gekennzeichnet, daß man die Umsetzung in flüssiger Phase in einem Festbettreaktor in Gegenwart heterogener Katalysatoren, die unter den Reaktionsbedingungen keine löslichen Bestandteile aufweisen, durchführt.

2. Verfahren nach Anspruch 1, dadurch gekennzeichnet, daß man die Umsetzung bei einer Temperatur im Bereich von 140 bis 320°C durchführt.

3. Verfahren nach einem der Ansprüche 1 bis 2, dadurch gekennzeichnet, daß man Aminocarbonsäurenitrile der Formel
H₂N―(CH₂)ₘ―C≡N
wobei
m 3, 4, 5 oder 6 ist, einsetzt.

4. Verfahren nach Anspruch 3, dadurch gekennzeichnet, daß man als Aminocarbonsäurenitril 6-Aminocapronsäurenitril einsetzt.

5. Verfahren nach einem der Ansprüche 1 bis 4, dadurch gekennzeichnet, daß man eine 1 bis 50 gew.-%ige Lösung des Aminocarbonsäurenitrils in Wasser oder in Wasser/org. Lösungsmittel-Gemischen einsetzt.

## Claims

1. A process for preparing cyclic lactams by reacting amino carbonitriles with water in the presence of catalysts, wherein the reaction is carried out in liquid phase in a fixed bed reactor in the presence of heterogeneous catalysts which have no soluble constituents under the reaction conditions.

2. A process as claimed in claim 1, wherein the reaction is carried out at from 140 to 320°C.

3. A process as claimed in either of claims 1 and 2, wherein amino carbonitriles of the formula
H₂N―(CH₂)ₘ―C≡N
where m is 3, 4, 5 or 6 are employed.

4. A process as claimed in claim 3, wherein 6-aminocapronitrile is employed as amino carbonitrile.

5. A process as claimed in any of claims 1 to 4, wherein a 1-50% by weight solution of the amino carbonitrile in water or in water/org. solvent mixtures is employed.

## Revendications

1. Procédé de préparation de lactames cycliques par réaction de nitriles d'acides aminocarboxyliques avec de l'eau, en présence de catalyseurs, caractérisé en ce qu'on effectue la réaction en phase liquide dans un réacteur à lit fixe en présence de catalyseurs hétérogènes qui, dans les conditions réactionnelles, ne présentent pas de composants solubles.

2. Procédé suivant la revendication 1, caractérisé en ce qu'on effectue la réaction à une température de l'ordre de 140 à 320°C.

3. Procédé suivant l'une des revendications 1 et 2, caractérisé en ce qu'on met en oeuvre des nitriles d'acides aminocarboxyliques de la formule :
H₂N-(CH₂)ₘ-C≡N
où m est égal à 3, 4, 5 ou 6.

4. Procédé suivant la revendication 3, caractérisé en ce que, comme nitrile d'acide aminocarboxylique, on met en oeuvre du nitrile d'acide 6-aminocaproïque.

5. Procédé suivant l'une des revendications 1 à 4, caractérisé en ce qu'on met en oeuvre une solution à 1 jusqu'à 50% en poids du nitrile d'acide aminocarboxylique dans de l'eau ou dans des mélanges d'eau/solvant organique.
